# EUROPÄISCHE PATENTSCHRIFT

(11) **EP 2 181 084 B1**
(45) Veröffentlichungstag und Bekanntmachung des Hinweises auf die Patenterteilung: **01.07.2015**
(21) Anmeldenummer: 08786173.8
(22) Anmeldetag: 16.07.2008
(51) Int. Cl.: C07C 29/17, C07C 35/12

(54) **VERFAHREN ZUR HERSTELLUNG VON MENTHOL DURCH HYDRIERUNG VON ISOPULEGOL**
PROCESS FOR PREPARATION OF MENTHOL BY HYDROGENATION OF ISOPULEGOL
PROCÉDÉ POUR OBTENIR DU MENTHOL PAR HYDROGÉNATION D'ISOPULÉGOL

(30) Priorität: 23.07.2007 EP 07112951
(43) Veröffentlichungstag der Anmeldung: 05.05.2010
(73) Patentinhaber: BASF SE, 67056 Ludwigshafen (DE)
(72) Erfinder: GRALLA, Gabriele, 68161 Mannheim (DE); HEYDRICH, Gunnar, 67117 Limburgerhof (DE); BERGNER, Eike Johannes, 69493 Hirschberg (DE); EBEL, Klaus, 68623 Lampertheim (DE)
(86) Internationale Anmeldenummer: PCT/EP2008/059277
(87) Internationale Veröffentlichungsnummer: WO 2009/013192

(56) Entgegenhaltungen:
- EP-A- 0 394 842
- DE-A1- 10 239 274

## Beschreibung

Die vorliegende Erfindung betrifft ein Verfahren zur Herstellung von racemischem oder optisch aktivem Menthol durch katalytische Hydrierung von racemischem oder optisch aktivem Isopulegol in Gegenwart von Wasserstoff und Katalysatoren, die Nickel-, Kupfer-, Zirkon- und Molybdän-haltige Verbindungen umfassen. Die vorliegende Erfindung betrifft speziell ein entsprechendes Verfahren zur kontinuierlichen katalytischen Hydrierung von L-Isopulegol zu L-Menthol.

Menthol, speziell das natürlich vorkommende L-Menthol stellt aufgrund seines minzartigen Aromas sowie seiner kühlenden und erfrischenden Eigenschaften eine der weltweit wichtigsten Aromachemikalien dar und wird in erheblichem Umfang zur Aromatisierung von Konsumgütern aller Art eingesetzt.

L-Menthol kann aus natürlichen Quellen, beispielsweise durch Kristallisation aus Pfefferminzöl oder auch durch synthetische Verfahren gewonnen werden. Nach dem Stand der Technik kann Menthol beispielsweise durch dearomatisierende Hydrierung von Thymol gewonnen werden, wobei es üblicherweise in Form eines Gemisches der Diastereomeren Menthol, Neo-Menthol, Iso-Menthol und Neoiso-Menthol anfällt, aus dem es durch weitere Verfahrensschritte isoliert werden muss.

Die DE 577 036 offenbart ein Verfahren zur Herstellung von synthetischem Menthol durch Hydrierung von Thymol. Als geeignete Katalysatoren werden Nickel-, Nickel/Kupfer- und Kobaltkatalysatoren beschrieben.

Spezielle Nickel-Katalysatoren wurden auch zur katalytischen Hydrierung von Piperitol zu Menthol eingesetzt, wie in der GB 1,503,723 beschrieben.

Die EP 1 532 091 offenbart ein Verfahren zur Herstellung von racemischem Menthol durch katalytische Hydrierung von Isopulegol, das in Form eines Diastereomerengemisches von 70,1% Isopulegol, 18,1% Neo-Isopulegol, 6,8% Iso-Isopulegol und 2,6% Neoiso-Isopulegol eingesetzt wurde. Als Katalysator wurde mit Eisen und Chrom dotiertes Raney-Nickel eingesetzt. Man erhielt Menthol in Form eines Gemisches der möglichen Diastereomeren, das zu 61,4% aus Menthol und zu 35,6% aus den weiteren Diastereomeren des Menthols bestand.

Einen weiteren Zugang zum Menthol stellen Verfahren zur diastereoselektiven Cyclisierung von Citronellal zum Isopulegol dar, wie sie beispielsweise in der WO 2006/092433 beschrieben ist. Das so erhaltene Isopulegol kann dann in einem weiteren Schritt zum Menthol hydriert werden.

R.H. Pickard et al. beschreiben in J. Chem. Soc. 1920, 1248 bis 1263 die Herstellung von L-Menthol durch katalytische Hydrierung von L-Isopulegol in Gegenwart von kolloidalem Palladium.

B. Dudley Sully et al. beschreiben in P.& E.O.R. 1068, 235 bis 366 die Herstellung von L-Menthol durch Hydrierung von L-Isopulegol in Gegenwart von Raney-Nickel bei einer Temperatur von 120°C.

Die EP 1 053 974 offenbart ein Verfahren zur katalytischen Hydrierung von Isopulegol zu Menthol in Gegenwart eines Katalysators von 5% Palladium auf Kohle bei einem Wasserstoffdruck von 5 bar.

Die EP 0 394 842 betrifft Katalysatoren für die Hydrierung aliphatischer ungesättigter Verbindungen, die Nickel und Kupfer enthalten und gekennzeichnet sind durch einen Gehalt von 20 bis 75 Gew.% Nickeloxid, 10 bis 75 Gew.% Zirkoniumdioxid und 5 bis 50 Gew.% Kupferoxid, jeweils bezogen auf den oxidischen, nicht reduzierten Katalysators. Als Substrate werden beispielhaft angegeben: Butin-2-diol-1,4, Buten-2-diol-1,4 und 2-Ethylhexen-2-al.

Ausgehend von diesem Stand der Technik bestand die Aufgabe der vorliegenden Erfindung darin, ein Verfahren bereitzustellen, das die Herstellung von im Wesentlichen diastereomerenreinem Menthol durch Hydrierung von im Wesentlichen diastereomerenreinem Isopulegol ermöglicht. Das Verfahren soll in technischen Maßstab auf verfahrenstechnisch gut handhabbare Weise durchführbar sein und in hoher chemischer Ausbeute unter Einsatz wohlfeiler Katalysatoren oder Reagenzien zum gewünschten Produkt führen. Dabei soll die Bildung von Diastereomeren des Menthols so weit als möglich vermieden werden. Darüber hinaus soll die Bildung von unerwünschtem Menthon bzw. Isomenthon und Neoiso-Menthol weitestgehend vermieden werden.

Die Aufgabe wurde erfindungsgemäß gelöst durch die Bereitstellung eines Verfahrens zur Herstellung von racemischem oder optisch aktivem Menthol der Formel (I) 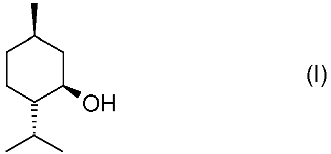 durch katalytische Hydrierung von racemischem oder optisch aktivem Isopulegol der Formel (II) 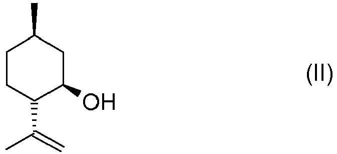 in Gegenwart von Wasserstoff und einem Katalysator, umfassend
- 30 bis 70 Gew.-% sauerstoffhaltige Verbindungen des Nickels, berechnet als NiO,
- 15 bis 45 Gew.-% sauerstoffhaltige Verbindungen des Zirkons, berechnet als ZrO₂,
- 5 bis 30 Gew.-% sauerstoffhaltige Verbindungen des Kupfers, berechnet als CuO und
- 0,1 bis 10 Gew.-% sauerstoffhaltige Verbindungen des Molybdäns, berechnet als MoO₃,
wobei sich die Angaben in Gew.-% auf den trockenen, nicht reduzierten Katalysator beziehen.

Als Ausgangsstoff zur Durchführung des erfindungsgemäßen Verfahrens eignet sich racemisches oder optisch aktives Isopulegol der Formel (II), wobei im Prinzip Isopulegol jeden Reinheitsgrades eingesetzt werden kann. Das erfindungsgemäße Verfahren eignet sich jedoch bevorzugt zur Umsetzung von Isopulegol hoher Reinheit, d.h. von Isopulegol mit einer Reinheit von 80 Gew.-% oder darüber, bevorzugt von 90 Gew.-% oder darüber. Insbesondere als Ausgangsstoff zur Durchführung des erfindungsgemäßen Verfahrens geeignet ist solches Isopulegol mit einer chemischen Reinheit von 97 Gew.-% oder darüber, bevorzugt von 98 bis 100 Gew.-%, besonders bevorzugt von 98,5 bis 99,9 Gew.-%, ganz besonders bevorzugt von mindestens 99 bis 99,9 Gew.-%. Dabei umfasst der Begriff chemische Reinheit auch die Diastereomerenreinheit des eingesetzten Isopulegols bezüglich der Diastereomere Neoiso-Isopulegol der Formel (III), Neo-Isopulegol der Formel (IV) und Iso-Isopulegol der Formel (V).

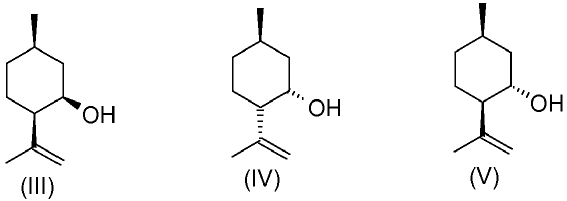

Dementsprechend weist ein als Ausgangsstoff zur Durchführung des erfindungsgemäßen Verfahrens besonders bevorzugtes Isopulegol eine wie vorstehend beschriebene Diastereomerenreinheit von 97 Gew.-% oder darüber, bevorzugt von 98 bis 100 Gew.-%, besonders bevorzugt von 98,5 bis 99,9 Gew.-% und ganz besonders bevorzugt von mindestens 99 bis 99,9 Gew.-% auf. Dabei können die genannten Formeln wie alle im Rahmen der vorliegenden Erfindung abgebildeten Formeln jeweils für beide Enantiomere (bzw. Gemische derselben) stehen und dienen zur Verdeutlichung der relativen Konfiguration der stereogenen Zentren.

Isopulegol kann erfindungsgemäß in racemischer oder nicht-racemischer, d.h. optisch aktiver Form eingesetzt werden. Bei Einsatz von racemischem Isopulegol der Formel (II) erhält man erfindungsgemäß racemisches Menthol der Formel (I). Bei Einsatz von optisch aktivem Isopulegol der Formel (II) erhält man entsprechend optisch aktives Menthol der Formel (I). Setzt man Isopulegol in optisch aktiver Form ein, sind erfindungsgemäß bevorzugt solche Gemische, die zum überwiegenden Teil das Enantiomer L-Isopulegol, wie es in seiner absoluten Konfiguration beispielhaft in Formel (II) wiedergegeben ist, enthalten. Erfindungsgemäß bevorzugt setzt man Isopulegol, d.h. D- oder bevorzugt L-Isopulegol mit einem Enantiomerenüberschuss (ee) von 80% ee oder darüber bevorzugt von 85 oder besser von 90% ee oder darüber, besonders bevorzugt von 95 bis 100% ee, ganz besonders bevorzugt von 96 bis 99,9% ee, weiterhin bevorzugt von 97 bis 99,8% ee, noch mehr bevorzugt von 98 bis 99,7% ee und insbesondere bevorzugt von 98,5 bis 99,6% ee ein. Ausgehend von L-Isopulegol in optisch aktiver Form erhält man in erfindungsgemäßer Weise L-Menthol in optisch aktiver Form.

Das erfindungsgemäße Verfahren wird in Gegenwart von Wasserstoff und in Gegenwart eines heterogenen Katalysators durchgeführt, wobei der einzusetzende heterogene Katalysator 30 bis 70 Gew.-%, bevorzugt 40 bis 60 Gew.-% sauerstoffhaltige Verbindungen des Nickels, berechnet als NiO, 15 bis 45 Gew.-%, bevorzugt 20 bis 40 Gew.-% sauerstoffhaltige Verbindungen des Zirkons, berechnet als ZrO₂, 5 bis 30 Gew.-%, bevorzugt 10 bis 25 Gew.-% sauerstoffhaltige Verbindungen des Kupfers, berechnet als CuO und 0,1 bis 10 Gew.-%, bevorzugt 0,5 bis 5 Gew.-% sauerstoffhaltige Verbindungen des Molybdäns, berechnet als MoO₃ gegebenenfalls neben weiteren Komponenten in einer Menge von 0 bis 10 Gew.-%, bevorzugt 0 bis 5 Gew.-% wie beispielsweise Graphit enthält. Dabei beziehen sich die Angaben in Gew.-% auf den trockenen, nicht reduzierten Katalysator.

Da sich die Konzentrationsangaben jeweils - falls nicht anders angegeben - auf die katalytisch aktive Masse des Katalysators beziehen, wird die katalytisch aktive Masse des Katalysators im folgenden als die Summe der Massen der katalytisch aktiven Bestandteile Zirkonium, Nickel, Kupfer und Molybdän im Katalysator, jeweils berechnet als ZrO₂, NiO, CuO bzw. MoO₃, nach dessen letzter Wärmebehandlung und vor dessen Reduktion mit Wasserstoff, definiert.

Im Rahmen einer bevorzugten Ausführungsform setzt man zur Durchführung des erfindungsgemäßen Verfahrens solche Katalysatoren ein, umfassend
- 45 bis 55 Gew.-% sauerstoffhaltige Verbindungen des Nickels, berechnet als NiO,
- 25 bis 35 Gew.-% sauerstoffhaltige Verbindungen des Zirkons, berechnet als ZrO₂,
- 5 bis 20 Gew.-% sauerstoffhaltige Verbindungen des Kupfers, berechnet als CuO,
- 1 bis 3 Gew.-% sauerstoffhaltige Verbindungen des Molybdäns, berechnet als MoO₃ und
- 0 bis 5 Gew.-% weiterer Komponenten,
wobei sich die Angaben in Gew.-% zu 100 Gew.-% ergänzen und sich auf den trockenen, nicht reduzierten Katalysator beziehen. Erfindungsgemäß insbesondere bevorzugt sind solche Katalysatoren, die aus den vorstehend genannten Komponenten in den ebenfalls vorstehend genannten Gewichtsanteilen bestehen.

Ein zum Einsatz im Rahmen des erfindungsgemäßen Verfahrens insbesondere bevorzugter Katalysator besteht zu 49 bis 53 Gew.-% aus NiO, zu 15 bis 19 Gew.-% aus CuO, zu 28 bis 32 Gew.-% aus ZrO₂ und zu 1 bis 2 Gew.-% aus MoO₃ sowie gegebenenfalls zu 0 bis 3 Gew.-% aus weiteren Komponenten wie beispielsweise Graphit, wobei sich die jeweils gewählten Gewichtsanteile der einzelnen Komponenten auf den trockenen, nicht reduzierten Katalysator beziehen und zu 100 Gew.-% ergänzen. Derartige Katalysatoren sind bekannt und können beispielsweise wie in der EP 0 696 572 beschrieben hergestellt werden.

Die erfindungsgemäß einsetzbaren Katalysatoren können z.B. durch Einsatz von Fällungsmethoden hergestellt werden. So können sie beispielsweise durch eine gemeinsame Fällung der Nickel- und Kupferkomponenten aus einer diese Elemente enthaltenden, wässrigen Salzlösung mittels Mineralbasen in Gegenwart einer Aufschlämmung einer schwerlöslichen, sauerstoffhaltigen Zirkoniumverbindung und anschließendes Waschen, Trocknen und Calcinieren des erhaltenen Präzipitats erhalten werden. Als schwerlösliche, sauerstoffhaltige Zirkoniumverbindungen können beispielsweise Zirkoniumdioxid, Zirkoniumoxidhydrat, Zirkoniumphosphate, -borate und -silikate Verwendung finden. Die Aufschlämmungen der schwerlöslichen Zirkoniumverbindungen können durch Suspendieren feinkörniger Pulver dieser Verbindungen in Wasser unter kräftigem Rühren hergestellt werden. Vorteilhaft werden diese Aufschlämmungen durch Ausfällen der schwerlöslichen Zirkoniumverbindungen aus wässrigen Zirkoniumsalzlösungen mittels Mineralbasen erhalten.

Bevorzugt werden die erfindungsgemäß einsetzbaren Katalysatoren über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Mineralbase, insbesondere einer Alkalimetallbase - beispielsweise Natriumcarbonat, Natriumhydroxid, Kaliumcarbonat oder Kaliumhydroxid - versetzt, bis die Fällung vollständig ist. Die Art der verwendeten Salze ist im allgemeinen nicht kritisch - da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Erfindungsgemäß einsetzbare Katalysatoren mit besonders vorteilhaften Eigenschaften sind dadurch erhältlich, dass man einen Teil der Zirkoniumkomponente des Katalysators, zweckmäßigerweise aus einer wässrigen Zirkoniumsalzlösung, separat in einer Fällungsapparatur durch Zugabe wässriger Mineralbasen fällt. Auf das so erhaltene, vorzugsweise frisch gefällte Zirkoniumoxid-Hydrat, kann dann der restliche Teil der Zirkoniumkomponente des Katalysators zusammen mit den anderen katalytisch aktiven Komponenten in einer Mischfällung gefällt werden, wie oben beschrieben wurde. Dabei erweist es sich in der Regel als besonders zweckmäßig 10 bis 80 Gew.-%, vorzugsweise 30 bis 70 Gew.-% und insbesondere 40 bis 60 Gew.-% der Gesamtzirkoniummenge der katalytisch aktiven Masse vorzufällen.

Die bei diesen Fällungsreaktionen erhaltenen Niederschläge sind im allgemeinen chemisch uneinheitlich und bestehen u.a. aus Mischungen der Oxide, Oxidhydrate, Hydroxide, Carbonate und unlöslichen und basischen Salze der genannten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge können wie üblich zu den erfindungsgemäß einsetzbaren Katalysatoren weiterverarbeitet werden. Nach dem Waschen werden sie im allgemeinen bei 80 bis 200°C, vorzugsweise bei 100 bis 150°C, getrocknet und danach calciniert. Die Calcinierung wird im allgemeinen bei Temperaturen zwischen 300 und 800°C, vorzugsweise bei 400 bis 600°C, insbesondere bei 450 bis 550°C ausgeführt.

Nach der Calcinierung wird der Katalysator zweckmäßigerweise konditioniert, sei es, dass man ihn durch Vermahlen auf eine bestimmte Korngröße einstellt oder dass man ihn nach seiner Vermahlung mit Formhilfsmitteln wie Graphit oder Stearinsäure vermischt, mittels einer Tablettenpresse zu Formlingen verpresst und tempert. Die Temperaturen entsprechen dabei im allgemeinen den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Die auf diese Weise hergestellten Katalysatoren können als solche gelagert und eingesetzt werden. Vor ihrem Einsatz als Katalysatoren im Rahmen des erfindungsgemäßen Verfahrens werden sie üblicherweise vorreduziert. Sie können jedoch auch ohne Vorreduktion eingesetzt werden, wobei sie dann unter den Bedingungen der erfindungsgemäßen Hydrierung durch den im Reaktor vorhandenen Wasserstoff reduziert werden. Zur Vorreduktion werden die Katalysatoren im allgemeinen zunächst bei 150 bis 200°C über einen Zeitraum von 12 bis 20 Stunden einer Stickstoff-WasserstoffAtmosphäre ausgesetzt und anschließend noch bis zu ca. 24 Stunden bei 200 bis 300°C in einer Wasserstoffatmosphäre behandelt. Bei dieser Vorreduktion wird ein Teil der in den Katalysatoren vorliegenden sauerstoffhaltigen Metallverbindungen üblicherweise zu den entsprechenden Metallen reduziert, so dass diese gemeinsam mit den verschiedenartigen Sauerstoffverbindungen in der aktiven Form des Katalysators vorliegen.

Im allgemeinen werden die erfindungsgemäßen Katalysatoren bevorzugt in Form von Vollkatalysatoren eingesetzt. Mit dem Begriff "Vollkatalysator" wird ein Katalysator bezeichnet, der im Gegensatz zu einem Trägerkatalysator nur aus katalytisch aktiver Masse besteht. Vollkatalysatoren können dergestalt eingesetzt werden, dass man die katalytisch aktive, zu Pulver vermahlene Masse in das Reaktionsgefäß einbringt oder, dass man die katalytisch aktive Masse nach Mahlung, Vermischung mit Formhilfsmitteln, Formung und Temperung als Katalysatorformkörper - beispielsweise als Kugeln, Zylinder, Tabletten, Ringe, Spiralen, Stränge und dergleichen mehr - im Reaktor anordnet.

Im Rahmen einer bevorzugten Ausführungsform des erfindungsgemäßen Hydrierverfahrens setzt man den gewählten heterogenen Katalysator in Form eines Festbettkatalysators ein.

Zur Durchführung des erfindungsgemäßen Verfahrens bringt man den wie vorstehend beschriebenen Ausgangsstoff Isopulegol mit Wasserstoff und dem gewählten Katalysator in Kontakt. Der Wasserstoff kann dabei in unverdünnter Form, üblicherweise in einer Reinheit von etwa 99,9 Vol.-% oder in verdünnter Form, d.h. in Form von Gemischen mit inerten Gasen wie beispielsweise Stickstoff oder Argon, eingesetzt werden. Bevorzugt setzt man Wasserstoff in unverdünnter Form ein.

Die Umsetzung kann mit gutem Erfolg ohne Zusatz von Lösungsmittel oder in Gegenwart von unter den Reaktionsbedingungen inerten organischen Lösungsmitteln wie beispielsweise Methanol, Ethanol, Isopropanol, Hexan, Heptan, Cyclohexan und dergleichen mehrdurchgeführt werden. Bevorzugt führt man die Reaktion ohne Zusatz von Lösungsmittel durch.

Die erfindungsgemäße Hydrierung von Isopulegol kann bei einem Wasserstoffdruck (absolut) im Bereich von 1 bis 200 bar, bevorzugt von 2 oder besser von 3 bis 200 bar, besonders bevorzugt von 4 oder 5 bis 150 bar, besonders bevorzugt von 5 bis 100 bar und ganz besonders bevorzugt im Bereich von 5 bis 50 bar durchgeführt werden. Als Reaktionstemperatur zur Durchführung der erfindungsgemäßen Hydrierung wählt man vorteilhaft eine Temperatur im Bereich von 20 bis 150°C, bevorzugt von 40 bis 130°C, besonders bevorzugt von 60 bis 110°C und ganz besonders bevorzugt von 70 bis 100°C.

Praktisch geht man bei der Durchführung im allgemeinen so vor, dass man dem Katalysator, der sich üblicherweise in einem bevorzugt von außen beheizten Festbettreaktor wie beispielsweise einem Rohrreaktor, Autoklaven oder Rohrbündelreaktor befindet, bei der gewünschten Reaktionstemperatur und dem gewünschten Druck das umzusetzende Isopulegol zuführt. Dabei belastet man den Katalysator im Allgemeinen mit 0,1 bis 1,0, bevorzugt mit 0,1 bis 0,6 und besonders bevorzugt mit 0,2 bis 0,4 kg Isopulegol pro kg Katalysator und pro Stunde. Hierbei kann es zweckmäßig sein, das einzusetzende Isopulegol bereits vor der Zuführung in das Reaktionsgefäß bzw. den Reaktor zu erwärmen und zwar bevorzugt auf die Reaktionstemperatur.

Der Reaktor kann sowohl in der Sumpf- als auch in der Rieselfahrweise betrieben werden, d.h. man kann die Ausgangsstoffe sowohl von unten nach oben als auch von oben nach unten durch den Reaktor leiten. Das erfindungsgemäße Hydrierverfahren kann sowohl diskontinuierlich als auch kontinuierlich durchgeführt werden. In beiden Fällen kann nicht umgesetztes Edukt zusammen mit dem Wasserstoff im Kreis geführt werden.

Die erfindungsgemäße Hydrierung kann auch stufenweise in einer Kaskade von mehreren, d.h. 2 bis in der Regel 4, bevorzugt 2 oder 3 und insbesondere bevorzugt in zwei hintereinander geschalteten Reaktoren, bevorzugt Festbettreaktoren durchgeführt werden. Dabei wird in dem ersten, üblicherweise als Hauptreaktor bezeichneten Reaktor unter den vorstehend beschriebenen Reaktionsbedingungen der Hauptumsatz der Reaktion erzielt und das erhaltene Rohprodukt einem zweiten, üblicherweise als Nachreaktor bezeichneten Reaktor zugeführt, in dem das noch nicht umgesetzte Ausgangsmaterial in erfindungsgemäßer Weise zumindest weitgehend in L-Menthol überführt wird. Dabei können die Reaktionsbedingungen unabhängig voneinander vorzugsweise in den vorstehend genannten Bereichen gewählt werden.

Das erfindungsgemäße Verfahren kann diskontinuierlich, halb- oder vollkontinuierlich durchgeführt werden. Bevorzugt führt man das Verfahren kontinuierlich, insbesondere vollkontinuierlich durch, wobei die Ausgangsstoffe kontinuierlich in den Reaktor eingetragen und das erhaltenen Reaktionsgemisch bzw. Reaktionsprodukt kontinuierlich aus dem Reaktor ausgetragen werden. Es hat sich weiterhin als vorteilhaft erwiesen, aufgrund der Lage des Schmelzpunktes des erfindungsgemäßen Reaktionsproduktes Menthol, speziell L-Menthol für eine Beheizung der eingesetzten Transportleitungen zu sorgen.

Das erfindungsgemäße Verfahren erlaubt die Herstellung von Menthol durch katalytische Hydrierung von Isopulegol, wobei es üblicherweise nur in geringem Ausmaß zur Bildung von unerwünschten Diastereomeren des Menthols kommt. Das erfindungsgemäße Verfahren liefert dementsprechend, bei Einsatz von Isopulegol mit einer entsprechenden Reinheit, Menthol der Formel (I) in einer chemischen Reinheit von 97 Gew.-% oder darüber, bevorzugt von 98 bis 100 Gew.-%, besonders bevorzugt von 98,5 bis 99,9 Gew.-%, ganz besonders bevorzugt von mindestens 99 bis 99,9 Gew.-%. Dabei umfasst der Begriff chemische Reinheit auch die Diastereomerenreinheit des erhaltenen Menthols bezüglich der Diastereomere Neoiso-Menthol der Formel (VI), Neo-Menthol der Formel (VII) und Iso-Menthol der Formel (VIII). Dementsprechend liefert das erfindungsgemäße Verfahren im Rahmen bevorzugt Menthol mit einer Diastereomerenreinheit von 97 Gew.-% oder darüber, bevorzugt von 98 bis 100 Gew.-%, besonders bevorzugt von 98,5 bis 99,9 Gew.-% und ganz besonders bevorzugt von mindestens 99 bis 99,9 Gew.-%.

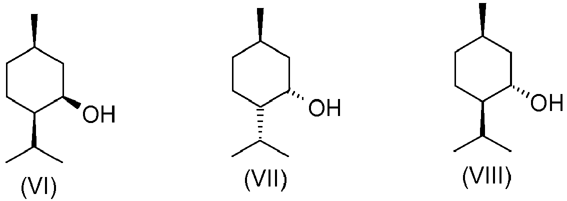

Setzt man Isopulegol in optisch aktiver Form, erfindungsgemäß bevorzugt solche Gemische, die zum überwiegenden Teil das Enantiomer L-Isopulegol enthalten ein, erhält man als erfindungsgemäßes Verfahrensprodukt in der Regel Menthol in optisch aktiver Form, bevorzugt in Form des (-)- bzw-L-Menthols. Die erfindungsgemäße Hydrierung verläuft im Regelfall weitgehend ohne nennenswerte Racemisierung des eingesetzten Materials. Demnach erhält man, in Abhängigkeit vom Enantiomerenüberschuss des eingesetzten optisch aktiven Isopulegols optisch aktives, bevorzugt bei Einsatz von L-Ispulegol L-Menthol mit einem Enatiomerenüberschuss (ee) von 80% ee oder darüber, bevorzugt von 85 oder 90% ee oder darüber, besonders bevorzugt von 95 bis 100% ee, besonders bevorzugt von 96 bis 99,9% ee, ganz besonders bevorzugt von 97 bis 99,8% ee, noch mehr bevorzugt von 98 bis 99,7% ee und insbesondere bevorzugt von 98,5 bis 99,6% ee ein.

Das erfindungsgemäß erhaltene Menthol zeichnet sich darüber hinaus durch einen besonders geringen Gehalt der unerwünschten Nebenprodukte Menthon der Formel (IX) und Isomenthon der Formel (X) und Neoiso-Menthol der Formel (VI) aus.

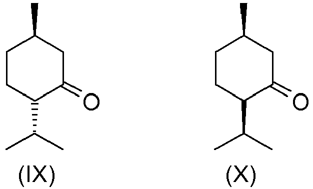

Diese Nebenprodukte werden im Regelfall im Rahmen des erfindungsgemäßen Verfahrens nur in einem auf die Menge an erhaltenem Menthol bezogenen Anteil von bis zu 0,5 Gew.-%, bevorzugt 0,4 Gew.-%, besonders bevorzugt 0,3 Gew.-%, insbesondere 0,2 Gew.-% und ganz besonders bevorzugt 0,1 bis 0 Gew.-% erhalten.

### Beispiele:

Die folgenden Beispiele dienen der Erläuterung der Erfindung, ohne sie in irgendeiner Weise zu beschränken:Gaschromatographische Analysen wurden nach der folgenden Methode durchgeführt:
50 m CP-WAX, ID.: 0,32 mm, FD.: 1,2 µm; 80 °C, 3 °C/min -200 °C, 10 °C/min auf 230 °C; t_{R} (Menthon): 26,9; t_{R} (Menthon): 28,1; t_{R} (Isopulegol): 30,7; t_{R} (Neo-Menthol): 31,2; t_{R} (Neoiso-Menthol): 32,6; t_{R} (Menthol): 32,7; t_{R} (Iso-Menthol): 34,1.

Das eingesetzte Isopulegol wurde gaschromatographisch folgendermaßen analysiert: 50 m CP-WAX, ID.: 0,32 mm, FD.: 1,2 µm; 80 °C, 3 °C/min -200 °C, 15 °C/min auf 250 °C; t_{R} (Citronellal): 21,6; t_{R} (Isopulegol-Isomer): 25,4; t_{R} (Isopulegol): 25,9; t_{R} (Citronellol): 32,7.

### Beispiel 1:

Es wurde eine Hydrierapparatur bestehend aus einem Hauptreaktor (HR) und einem Nachreaktor (NR) eingesetzt. Der Hauptreaktor wies 5 in Reihe geschaltete Röhren mit einem Innendurchmesser von 5 mm und einer Länge von 1,3 m auf, die mit 61 g (127 ml) eines Festbettkatalysator enthaltend 50 Gew.-% NiO, 17 Gew.-% CuO, 30,5 Gew.-% ZrO₂, 1,5 Gew.-% MoO₃ und 1 Gew.-% Graphit in Form von Tabletten mit einem Durchmesser und einer Höhe von jeweils von 3 mm gefüllt waren. Der Nachreaktor (Doppelmantel) bestand aus einem Rohr mit einem Innendurchmesser von 5 mm und einer Länge von 2,05 m, der mit 19 g des gleichen Katalysators gefüllt war.

Der im Hauptreaktor und Nachreaktor eingebaute Festbettkatalysator enthaltend 50 Gew.-% NiO, 17 Gew.-% CuO, 30,5 Gew.-% ZrO₂, 1,5 Gew.-% MoO₃ und 1 Gew.-% Graphit wurde nach folgender Vorschrift aktiviert. Die Reaktoren wurden mit 42 Nl/h Stickstoff und 1,2 Nl/h Wasserstoff drucklos auf 180 °C geheizt und 19 h bei diesen Bedingungen gehalten. Der Wasserstoff wurde von 1,2 auf 6,5 Nl/h erhöht und weitere 7,5 h bei einer Temperatur von 180 °C gehalten. Der Stickstoff-Zulauf wurde abgedreht und mit 6,5 Nl/h Wasserstoff 12 h bei 180 °C die Aktivierung fortgesetzt. Anschließend wurde der Wasserstoff-Zulauf abgedreht und der Stickstoff-Zulauf auf 6 Nl/h eingestellt. Die Reaktoren wurden auf eine Temperatur von 60 °C abgekühlt. Der Wasserstoff-Zulauf wurde auf 1,6 Nl/h reduziert und mit dem Isopulegol-Zulauf gestartet.

Mittels einer Kreiselpumpe wurde ein Umwälzkreislauf über den Hauptreaktor mit einer Rate von etwa 500 g/h bei einem Zulauf an L-Isopulegol von 24,5 g/h (Gesamtmenge 588 g) mit einer Reinheit von 99,9 Gew-% und 99,8% ee gepumpt. Der Wasserstoffdruck wurde konstant bei 40 bar gehalten. Der Hauptreaktor wurde mit einer Temperatur von 85°C und der Nachreaktor mit 75°C betrieben. Sämtliche Rohrleitungen wurden zur Vermeidung der Auskristallisation des enantiomerenreinen L-Menthols (Smp. 44°C) mit einer elektrischen Begleitheizung versehen. Man erhielt L-Menthol in einer Menge von 597 g entsprechend einer Rate von 24,9 g/h. Das so erhaltene L-Menthol (99,8% ee) wurde gaschromatographisch analysiert. Die chemische Reinheit des Austrags L-Menthol ist in Tabelle 1 zusammengestellt.

**Tabelle 1: GC-Analytik des Austrags L-Menthol (GC-FI.-%)**

| Menthon / Isomenthon | L-Menthol | Neo-Menthol | Neoiso-Menthol | Iso-Menthol | L-Isopulegol |
|---|---|---|---|---|---|
| 0 | 99,6 | 0,19 | 0 | 0 | 0,19 |

### Beispiel 2:

Beispiel 1 wurde unter Einsatz von L-Isopulegol mit einer Reinheit von 99,9 Gew.-% und 99,8% ee, das mit einer Rate von 12,6 g/h (Gesamtmenge 303 g) in den Reaktor eingetragen wurde bei einem Wasserstoffdruck von 40 bar wiederholt. Der Hauptreaktor (HR) wurde auf 80°C, der Nachreaktor auf 75°C temperiert. Man erhielt L-Menthol (99,8% ee) in einer Menge von 306 g entsprechend einer Rate von 12,8 g/h. Die chemische Reinheit des Austrags L-Menthol ist in Tabelle 2 zusammengestellt.

**Tabelle 2: GC-Analytik des Austrags L-Menthol (GC-FI.-%)**

| Menthon / Isomenthon | L-Menthol | Neo-Menthol | Neoiso-Menthol | Iso-Menthol | L-Isopulegol |
|---|---|---|---|---|---|
| 0 | 99,7 | 0,25 | 0 | 0 | 0 |

### Beispiel 3:

Beispiel 1 wurde unter Einsatz von L-Isopulegol mit einer Reinheit von 97,1 % und 84% ee, das mit einer Rate von 24,5 g/h (Gesamtmenge 466 g) in den Reaktor eingetragen wurde bei einem Wasserstoffdruck von 40 bar wiederholt. Der Hauptreaktor (HR) wurde auf 80°C, der Nachreaktor auf 70°C temperiert. Das eingesetzte L-Isopulegol wies die folgende Zusammensetzung auf: L-Isopulegol: 97,1 GC-Gew.-%, Citronellol: 0,05 GC-Gew.-%, Citronellal: 0,40 GC-Gew.-%, Isopulegol-Isomer 0,45 GC-Gew-%, Nebenkomponente: 0,34 GC-Gew.-%. Man erhielt L-Menthol (84% ee) in einer Menge von 468 g entsprechend einer Rate von 24,6 g/h. Die chemische Reinheit des Austrags L-Menthol ist in Tabelle 3 dargestellt.

**Tabelle 3: GC-Analytik des Austrags L-Menthol (GC-FI.-%)**

| Menthon / Isomenthon | L-Menthol | Neo-Menthol | Neoiso-Menthol | Iso-Menthol | L-Isopulegol | Neben-Komp. |
|---|---|---|---|---|---|---|
| 0,08/0 | 97,3 | 1,0 | 0,29 | 0,20 | 0,29 | 0,33 |

### Vergleichsbeispiel 1:

Es wurde eine Hydrierapparatur bestehend aus einem Hauptreaktor (HR) und einem Nachreaktor (NR) eingesetzt. Der Hauptreaktor wies 5 in Reihe geschaltete Röhren mit einem Innendurchmesser von 5 mm und einer Länge von 1,3 m auf, die mit 104 g (127 ml) eines Festbettkatalysator bestehend aus 0,47 Gew.-% Palladium auf einem γ-Al₂O₃-Träger in Form von Strängen mit einer Länge von 4 mm gefüllt waren. Der Nachreaktor (Doppelmantel) bestand aus einem Rohr mit einem Innendurchmesser von 5 mm und einer Länge von 1,9 m, das mit 27 g (35 ml) des gleichen Katalysators gefüllt war.

Mittels einer Kreiselpumpe wurde ein Umwälzkreislauf über der Hauptreaktor mit einer Rate von etwa 500 g/h bei einem Zulauf an L-Isopulegol von 24,5 g/h (Gesamtmenge 588 g) mit einer Reinheit von 99,8% und 99,8% ee bei einem konstanten Wasserstoffdruck von 30 bar betrieben. Der Hauptreaktor wurde mit einer Temperatur von 50°C und der Nachreaktor mit 60°C betrieben. Sämtliche Rohrleitungen wurden zur Vermeidung der Auskristallisation des enantiomerenreinen L-Menthols (Smp. 44°C) mit einer elektrischen Begleitheizung versehen. Man erhielt L-Menthol (99,8% ee) in einer Menge von 597 g entsprechend einer Rate von 24,9 g/h. Das so erhaltene Produkt wurde gaschromatographisch analysiert. Die Ergebnisse sind in Tabelle 4 zusammengestellt.

**Tabelle 4: GC-Analytik des erhaltenen L-Menthols (GC-FI.-%)**

| Menthon / Isomenthon | L-Menthol | Neo-Menthol | Neoiso-Menthol | Iso-Menthol | L-Isopulegol | Neben-Komp. |
|---|---|---|---|---|---|---|
| 0,64 / 0,56 | 97,5 | 0 | 0,66 | 0 | 0,29 | 0,10 |

## Patentansprüche

1. Verfahren zur Herstellung von racemischem oder optisch aktivem Menthol der Formel (I) 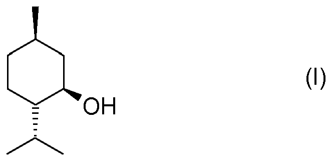 durch katalytische Hydrierung von racemischem oder optisch aktivem Isopulegol der Formel (II) 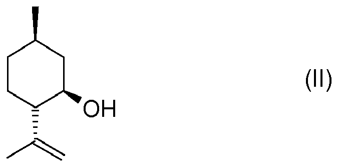 in Gegenwart von Wasserstoff und einem Katalysator, umfassend
- 30 bis 70 Gew.-% sauerstoffhaltige Verbindungen des Nickels, berechnet als NiO,
- 15 bis 45 Gew.-% sauerstoffhaltige Verbindungen des Zirkons, berechnet als ZrO₂,
- 5 bis 30 Gew.-% sauerstoffhaltige Verbindungen des Kupfers, berechnet als CuO und
- 0,1 bis 10 Gew.-% sauerstoffhaltige Verbindungen des Molybdäns, berechnet als MoO₃,
wobei sich die Angaben in Gew.-% auf den trockenen, nicht reduzierten Katalysator beziehen.

2. Verfahren nach Anspruch 1 zur Herstellung von L-Menthol ausgehend von L-Isopulegol.

3. Verfahren nach Anspruch 1 oder 2, **dadurch gekennzeichnet, dass** man einen Katalysator einsetzt, umfassend
- 45 bis 55 Gew.-% sauerstoffhaltige Verbindungen des Nickels, berechnet als NiO,
- 25 bis 35 Gew.-% sauerstoffhaltige Verbindungen des Zirkons, berechnet als ZrO₂,
- 5 bis 20 Gew.-% sauerstoffhaltige Verbindungen des Kupfers, berechnet als CuO,
- 1 bis 3 Gew.-% sauerstoffhaltige Verbindungen des Molybdäns, berechnet als MoO₃ und
- 0 bis 5 Gew.-% weiterer Komponenten,
wobei sich die Angaben in Gew.-% zu 100 Gew.-% ergänzen und sich auf den trockenen, nicht reduzierten Katalysator beziehen.

4. Verfahren nach einem der Ansprüche 1 bis 3, **dadurch gekennzeichnet, dass** man den Katalysator in Form eines Vollkatalysators einsetzt.

5. Verfahren nach einem der Ansprüche 1 bis 4, **dadurch gekennzeichnet, dass** man den Katalysator in Form eines Festbettkatalysators einsetzt.

6. Verfahren nach einem der Ansprüche 1 bis 5, **dadurch gekennzeichnet, dass** man Menthol der Formel (I) in einer Reinheit von mindestens 99 Gew.-% erhält.

7. Verfahren nach einem der Ansprüche 1 bis 6, **dadurch gekennzeichnet, dass** man Isopulegol der Formel (II) in einer Reinheit von mindestens 99 Gew.-% einsetzt.

8. Verfahren nach einem der Ansprüche 1 bis 7, **dadurch gekennzeichnet, dass** man die Hydrierung bei einem Wasserstoffdruck im Bereich von 5 bis 200 bar absolut durchführt.

9. Verfahren nach einem der Ansprüche 1 bis 8, **dadurch gekennzeichnet, dass** man die Hydrierung bei einer Temperatur im Bereich von 50 bis 130°C durchführt.

10. Verfahren nach einem der Ansprüche 1 bis 9, **dadurch gekennzeichnet, dass** man die Hydrierung stufenweise in einer Kaskade von Reaktoren durchführt.

11. Verfahren nach einem der Ansprüche 1 bis 10, **dadurch gekennzeichnet, dass** man die Hydrierung kontinuierlich durchführt.

12. Verfahren nach einem der Ansprüche 1 bis 11, **dadurch gekennzeichnet, dass** man es ohne Zusatz von Lösungsmitteln durchführt.

13. Verfahren nach einem der Ansprüche 1 bis 12, **dadurch gekennzeichnet, dass** man den Katalysator mit 0,1 bis 1,0 kg Isopulegol pro kg Katalysator und pro Stunde belastet.

## Claims

1. A process for preparing racemic or optically active menthol of the formula (I) 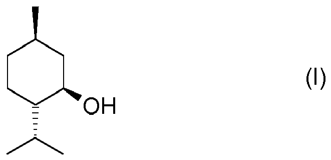 by catalytically hydrogenating racemic or optically active isopulegol of the formula (II) 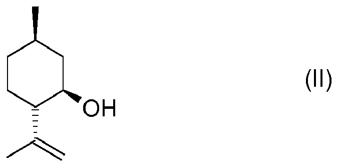 in the presence of hydrogen and a catalyst comprising
- from 30 to 70% by weight of oxygen compounds of nickel, calculated as NiO,
- from 15 to 45% by weight of oxygen compounds of zirconium, calculated as ZrO₂,
- from 5 to 30% by weight of oxygen compounds of copper, calculated as CuO and
- from 0.1 to 10% by weight of oxygen compounds of molybdenum, calculated as MoO₃,
where the figures in % by weight are based on the dry unreduced catalyst.

2. The process according to claim 1 for preparing L-menthol proceeding from L-isopulegol.

3. The process according to claim 1 or 2, wherein a catalyst comprising
- from 45 to 55% by weight of oxygen compounds of nickel, calculated as NiO,
- from 25 to 35% by weight of oxygen compounds of zirconium, calculated as ZrO₂,
- from 5 to 20% by weight of oxygen compounds of copper, calculated as CuO,
- from 1 to 3% by weight of oxygen compounds of molybdenum, calculated as MoO₃ and
- from 0 to 5% by weight of further components,
is used, where the figures in % by weight add up to 100% by weight and are based on the dry unreduced catalyst.

4. The process according to any one of claims 1 to 3, wherein the catalyst is used in the form of an unsupported catalyst.

5. The process according to any one of claims 1 to 4, wherein the catalyst is used in the form of a fixed bed catalyst.

6. The process according to any one of claims 1 to 5, wherein menthol of the formula (I) is obtained in a purity of at least 99% by weight.

7. The process according to any one of claims 1 to 6, wherein isopulegol of the formula (II) is used in a purity of at least 99% by weight.

8. The process according to any one of claims 1 to 7, wherein the hydrogenation is performed at a hydrogen pressure in the range from 5 to 200 bar absolute.

9. The process according to any one of claims 1 to 8, wherein the hydrogenation is performed at a temperature in the range from 50 to 130°C.

10. The process according to any one of claims 1 to 9, wherein the hydrogenation is performed stepwise in a cascade of reactors.

11. The process according to any one of claims 1 to 10, wherein the hydrogenation is performed continuously.

12. The process according to any one of claims 1 to 11, which is performed without addition of solvents.

13. The process according to any one of claims 1 to 12, wherein the catalyst hourly space velocity is from 0.1 to 1.0 kg of isopulegol per kg of catalyst and per hour.

## Revendications

1. Procédé pour la préparation de menthol racémique ou optiquement actif de formule (I) 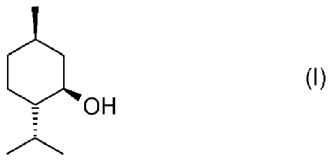 par hydrogénation catalytique d'isopulégol racémique ou optiquement actif de formule (II) 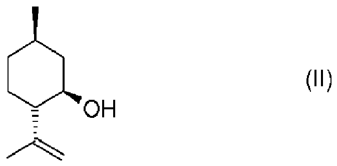 en présence d'hydrogène et d'un catalyseur, comprenant
- 30 à 70% en poids de composés oxygénés du nickel, calculés sous forme de NiO,
- 15 à 45% en poids de composés oxygénés du zirconium, calculés sous forme de ZrO₂,
- 5 à 30% en poids de composés oxygénés du cuivre, calculés sous forme de CuO, et
- 0,1 à 10% en poids de composés oxygénés du molybdène, calculés sous forme de MoO₃,
les indications en % en poids se rapportant au catalyseur sec, non réduit.

2. Procédé selon la revendication 1 pour la préparation de L-menthol partant de L-isopulégol.

3. Procédé selon la revendication 1 ou 2, **caractérisé en ce qu'**on utilise un catalyseur, comprenant
- 45 à 55% en poids de composés oxygénés du nickel, calculés sous forme de NiO,
- 25 à 35% en poids de composés oxygénés du zirconium, calculés sous forme de ZrO₂,
- 5 à 20% en poids de composés oxygénés du cuivre, calculés sous forme de CuO,
- 1 à 3% en poids de composés oxygénés du molybdène, calculés sous forme de MoO₃ et
- 0 à 5% en poids d'autres composants,
les indications en % en poids se complétant à 100% en poids et se rapportant au catalyseur sec, non réduit.

4. Procédé selon l'une quelconque des revendications 1 à 3, **caractérisé en ce qu'**on utilise le catalyseur sous forme d'un catalyseur plein.

5. Procédé selon l'une quelconque des revendications 1 à 4, **caractérisé en ce qu'**on utilise le catalyseur sous forme d'un catalyseur en lit fixe.

6. Procédé selon l'une quelconque des revendications 1 à 5, **caractérisé en ce qu'**on obtient le menthol de formule (I) à une pureté d'au moins 99% en poids.

7. Procédé selon l'une quelconque des revendications 1 à 6, **caractérisé en ce qu'**on utilise l'isopulégol de formule (II) à une pureté d'au moins 99% en poids.

8. Procédé selon l'une quelconque des revendications 1 à 7, **caractérisé en ce qu'**on réalise l'hydrogénation à une pression d'hydrogène dans la plage de 5 à 200 bars absolus.

9. Procédé selon l'une quelconque des revendications 1 à 8, **caractérisé en ce qu'**on réalise l'hydrogénation à une température dans la plage de 50 à 130°C.

10. Procédé selon l'une quelconque des revendications 1 à 9, **caractérisé en ce qu'**on réalise l'hydrogénation par étapes dans une cascade de réacteurs.

11. Procédé selon l'une quelconque des revendications 1 à 10, **caractérisé en ce qu'**on réalise l'hydrogénation en continu.

12. Procédé selon l'une quelconque des revendications 1 à 11, **caractérisé en ce qu'**on le réalise sans addition de solvants.

13. Procédé selon l'une quelconque des revendications 1 à 12, **caractérisé en ce qu'**on charge le catalyseur par 0,1 à 1,0 kg d'isopulégol par kg de catalyseur et par heure.
